# DEMANDE DE BREVET EUROPEEN

(11) **EP 1 491 197 A1**
(43) Date de publication de la demande: **29.12.2004**
(21) Numéro de dépôt: 03425418.5
(22) Date de dépôt: 26.06.2003
(51) Int. Cl.: A61K 31/551, A61K 35/78, A61P 37/04

(54) **Composition pharmaceutique à base de voacamine pour la stimulation et modulation du système immunitaire humain**

(71) Demandeur: Bertelli Motta, Guiseppe, 10060 Scalenghe (IT)
(72) Inventeur: Bertelli Motta, Giuseppe, 10060 Scalenghe (IT); Rossi, Silvio, 10040 Carignano (IT); Stauffer, Eric, 84040 Capriolo (IT)

(57) **Abrégé**

L'INVENTION CONCERNE LA VOACAMINE EN TANT QU'AGENT ACTIF DANS LA STIMULATION ET MODULATION DU SYSTEME IMMUNITAIRE HUMAIN, PRESENTANT UNE FORTE ACTIVITE QU'ELLE EXERCE SUR LE SYSTEME MEME.
Comme agent stimulant ayant pour principe actif de la voacamine, on peut utiliser un extrait basique de Peschiera, notamment de Peschiera fuchsiaefolia, ou de Voacanga africaine.

## Description

Souscrit Bertelli Motta Giuseppe né à Turin le 04/11/1943 et résident en Scalenghe, en collaboration avec Rossi Silvio, né à Poirino le 10/07/1946 résident à Carignano et à l'aide de Stauffer Eric né à Caprioli le 24/10/1964 et résident à Genève, et la contribution de l'Institut Supérieur de Santé Laboratoire de Recherche du Medicament Naturel de Rome pour l'extraction du principe active du Peschiera Fuchsiaefolia et de la plante Voacanga Africaine. En dirigeant et en coordonnant la recherche. La présente invention compte sur la voacamina et à son utilisation comme agent stimulant et modulant du système immunitaire humain, en présentant de forte activité. l'action qu'elle exerce sur le système même. La voacamina ou 12-methoxy-13-[(3-17-methoxy-17-oxovobasan-3-yi]ibogamine-18-carboxilato de méthyle, C43H52N405, formule représentée ici sous (I) un alcaloïde bis endolico dimero que présente des nombreuses espèce de plantes appartenant au genre Voacanga et Apocynacee.

Ce alcaloïde connu pour son activitè cytotossica (J.Nat. Prod., 1994, 57, 1517) par son activitè antibactérien, tant contre les en union synergique avec des parties, des extraits ou, extraits, o component de bactéries Gram + comme contre les bactéries Gram - (Phytochemistry, 1984.,23, 1771). L'objet de la présente invention est l'utilisation de la voacamina comme agent stimulant et modulant du système immunitaire en cas de HIV. Selon les inventeurs se met en évidence la force de la voacamina comme agent qui en stimulant et en modulant le système immunitaire a une augmentation des défenses contre les maladies en exerçant ainsi un marqué effet sur le bien-être de la personne.

Autre j'attends de l'invention concerne les agents stimulants contenus dans la voacamina en particulier l'extrait contiennent l'alcaloïde. L'invention rapporta donc aux extraits te bases-nous de plantes de la Voacanga, mais même aux extraits bases-nous du genre Peschiera, (ou Tabernaemontana) en particulier Peschiera Van Heurchii, Peschiera Campestris, Peschiera Affinis, Peschiera Laeta et en particulier du Peschiera Fuchsiaefolia.

Le Peschiera Fuchsiaefolia, une Apocynacea de sous espèce des Tabernaemontanoide une plante indigène de l'Amérique du Sud, en particulier du bassin amazonien. Appelle au Brésil. Leitero de Vaca. une plante infestant et comme tel est traité avec des pesticide. Un rapport récent explique même son emploi pour le sien activité neutraliser le poison de morsure de cobra (J. Venemous Âme Toxinns 1997.,3, 22). L'invention concerne même un extrait basique de Peschiera, en particulier Fuchsiaefolia et particularité un extrait basique des graines de la plante et de l'écorce du fût et ram au-delà de que des racines, riches en alcaloïdes tertiaire.

Constatait qui l'extrait basique consistant dans la fraction riche en alcaloïdes terziari de l'écorce des racines du Peschiera Fuchsiaefolia a une activité major au voacamina seule, dans cet extrait prouve la présence de peverine, le 16-epi- affisine, l'affinisina, vocamidina, les 16 dacarbomethoxyvoacamine, le nb- Demethyllvoacamine, le tabernamina, l'ervahanina à, le coronadina, le voachalotina, le heyneianina, les voacristine, le conopharyngina, le vobasina et le voacangina. (Tu vois les formules II ; III ; IV).

Le tabernamina, l'ervahanine à, le vobasina, les conopharygine, le heyneanina, le voacristina et le conoradina sont extraits pour le première tourne de cette plante avec l'aide de la voacamina ou d'autres extraits bases-nous de plantes en contenant voacamina possible fabriquer des déesses médicaments en ayant la présence des alcaloïdes sous forme de base ou de sels physiologiquement acceptables, incorporés dans un supporte des ails excipients. Autre j'attends de l'invention est une procédure d'isolement à partir du Peschiera en particulier du Peschiera Fuchsiaefolia, d'un extrait basique avec procédure pour isolation de le voacamina à partir de la plante même, ayant des vertus stimulantes le système immunitaire humain.

L'extrait en question peut être préparé pour le traitement pour un acide dilué d'un extrait sèche de Peshiera Fuchsiaefolia ou d'une partie seule de la plante pour extraire les principes bases-nous, suivis des alcalinisation de l'extrait aqueux des ainsi obtenu n à PH9 et de l'extraction de la fraction riche en alcaloïdes tertiaire avec un solvant organique, par exemple de dichloromethano.

L'isolation de la voacamina à partir de cette fraction riche en alcaloïdes tertiaire est réalisée pour distribution dans contre-courant dans une phase aqueuse et une phase organique en faisant varier le PH dans la phase aqueuse. Les voacamine et les mêmes extraits bases-nous qu'ils la contiennent peuvent être formulé en bas forme de médicaments, seconde les techniques galéniques classiques, par exemple sous forme d'injections, de sirop, de supposées, de comprimées, capsules. Ils s'utiliseront des excipients et des supports farmacologicamente acceptables, comme les sucres, le lactose, l'etc.

Dans les formulations pharmaceutiques le voacamina et les alcaloïdes qui éventuellement l'accompagnent pourront se trouver en forme libre ou comme sels physiologiquement acceptables comme i clofibrate, etc.

La formulation employée dans vivant comme j'emploie compassionnelle pour les enfants fournies de médecins opérants en Mozambique et en Benin, comme en Zambie il a eu comme sujet un extrait je sèche dans comprimées de 500 mg. Avec posologie de deux comprimées trois fois au giorno, (deux chaque quatre heures environ). Les analyse ematique ont été exécutés à l'Université de Milan de Recherche Immunologie dirigée de Prof. Mario Clerici. Les tests ont été exécuté sur des enfants de différents des ans d'âge, en phase terminale de la maladie HIV.

Les données plus importantes qui ont contrôle, sont données des cellules TH2, responsables de la défense d'agent pathogène extra-cellulaire.

Normalement les valeurs relatives à ces cellules dans la population africaine sont très élevées.

Ce facteur est donné de l'insuffisante hygiène de leurs conditions de vie qui soumet l'organisme et la sollicitation continue de la part de microbes et les bactéries présentes dans l'eau, dans la nourriture, dans l'air, etc. Cela signifie que les précurseurs des lymphocytes T se diversifient en TH2 à perte de la différenciation vers TH1, qui sont des cellules députées à la défense contre agent pathogènes intracellulaire (virus en particulier). Ce qui nous le remarquons la nette diminution des cellules th2 et t2, qui peut être vue positivement si compensée d'augmentation du th1. Les données relatives au th1 actives, sont contrôlables à travers la production de citochine qui effectivement subissent une contrôlable augmentation facteur très important, en outre est visible même une augmentation des lymphocytes T à action cytotoxique (Ctl - T 8), député même ils à la défense contre les agents pathogèneintracellulaire.

La différence entre CTL et le TH1 est-leur mécanisme d'action. CTL agissent directement sur les cellules infectées de virus, pendant que le TH1 elles agissent en activant des monolithes à action fagoteuse. Donc on peut affirmer que la thérapie a donné résultés, en stimulant le système immunitaire des patients et en particulier en exerçant la réaction des cellules députées à la défense contre les virus.

Dans le schéma PHÉNOTYPE, (%CELLULE POSITIVE) nous avons vu comme le T CD3 qui sont des lymphocytes T totaux, cellules de l'immunité spécifique présentent une positive augmentation (tu vois des Appendices 1-2-3). Dans le T ATT, lymphocytes T tu actives, la diminution positive des valeurs indique une diminution de la sollicitation virale.

Dans les lymphocytes B, qui sont des cellules de l'immunité spécifique, il ne résulte pas être vérifié un changement significatif pour cause de notre traitement. NK, (Natural Killer) sont les cellules de l'immunité naturelle. Même sur cette typologie de cellules il ne s'enregistre pas en changement significatif.

Sur les CD4DR2 qui est les lymphocytes TH tu actives, relevons une positive diminution des valeurs, un qu'il indique une diminution de la sollicitation virale, c'est-à-dire affaiblissement de l'action du Virus.

Sur les CD8DR2, les lymphocytes CTL tu actives, on obtient une positive diminution des valeurs, même dans ce cas il signifie une diminution de la sollicitation exercée du virus.

Le CD4, les lymphocytes TH totaux, le montrer une positive augmentation des valeurs, qu'il indique une reprise de la défense immunitaire de la part de ces cellules preplacées à l'immunité cellulaire.

Le CD8, les lymphocytes CTL totaux, subissent une positive augmentation des valeurs de référence qui même dans ce cas indique une augmentation de la défense immunitaire de la part de ces cellules preplacées de l'immunité cellulaire en signifiant une donnée positive sur l'action du principe active fourni.

Les CD4/CD ne présentent pas un changement significatif entre les deux temps, D'abord et après les fourniture.

Le CD4 NAUVE qui sont les cellules TH prêtes à intervenir, mais n'ancre pas activées de contacte avec le virus ou avec l'antigène ils ont subi une augmentation significative montrée dans le tableau.

Les valeurs indiquent vivacité du système immunitaire à intervenir. CD4 - Mem, ou TH Mémoire, est une partie des cellules qui ont réagi avec le virus et qu'on transforme en cellules prêtes à réagir opportunément à selon attaque du même virus. La positive diminution des valeurs indique une minore sollicitation virale, en outre TH Mémoire ne sont pas efficace contre HIV parce que le virus change et ces cellules ne sont pas en mesure de reconnaître selon attaque.

TH1 : pour des significatives analyse de l'action de ces cellules il est nécessaire visionnaire les valeurs des citochine.

TH2 : positive diminution des valeurs en faveur du TH1.

CD8 - NAIVE : CTL ne sont pas encore venus en contacte avec le virus et donc elles ne sont pas encore activées, mais prêtes à réagir.

La positive augmentation des valeurs signifie un majeur nombre de cellules prêtes à réagir, dû à une diminution d'attaque viral.

CD8 EFFEC : lorsque CTL sont actif est parce qu'ils sont stimulés de Virus. La positive diminution des valeurs représente une diminution d'attaque viral.

CD8 MEN : CTL de la mémoire. L'augmentation de leurs valeurs n'est pas significative.

CD8 R038 : CTL tu actives. La positive diminution de leurs valeurs va attribuer, au même motive de CTL EFFETTRICI :
CD14 CCR5 : des monolithes qui expriment un corecettore CCR5 où s'attaque un type de HIV (le récepteur véritable est le récepteur CD4 qui est exprimé de TH). Sur ce point l'interprétation de l'activité reste doute.
CD4 CCR5 : TH qui expriment le même corecettore.
CD8 CD95 : CTL sont en train de subir des apoptose.
CITOCHINE : sont produites du TH1. Les significatives valeurs exprimées dans les temps de culture sont appelées MED et PHA.
IFN - g IL2 : est des substances produites du TH1 qui en augmentent l'activité contre les virus. Leur positive augmentation des valeurs représente l'activité et l'efficacité du TH1.
TNF M : est la substance produite dans l'inflammation. On enregistre une brève augmentation des valeurs ; cela est négatif parce qu'il montre une légère augmentation d'inflammation pas nécessairement liée au virus HIV.

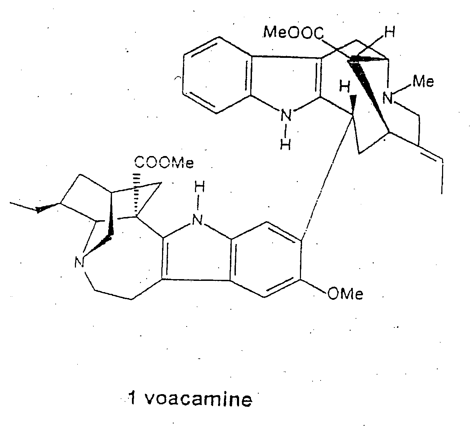

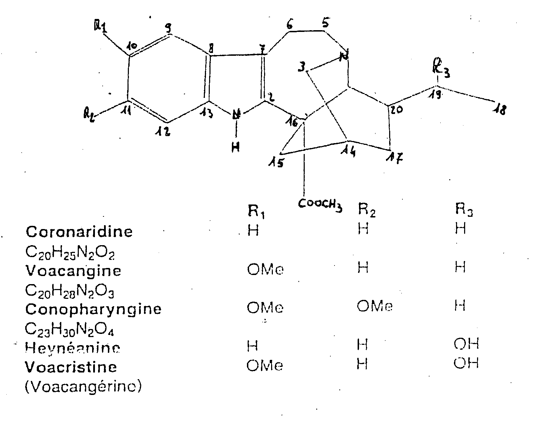

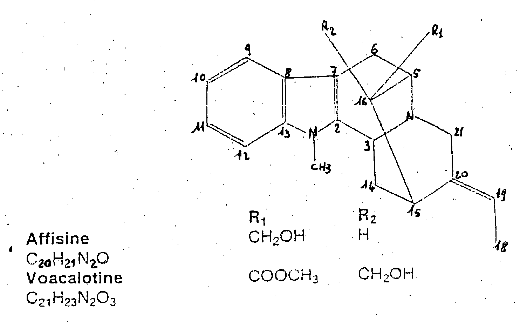

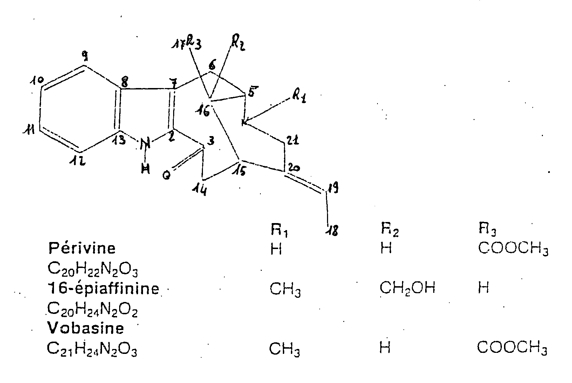

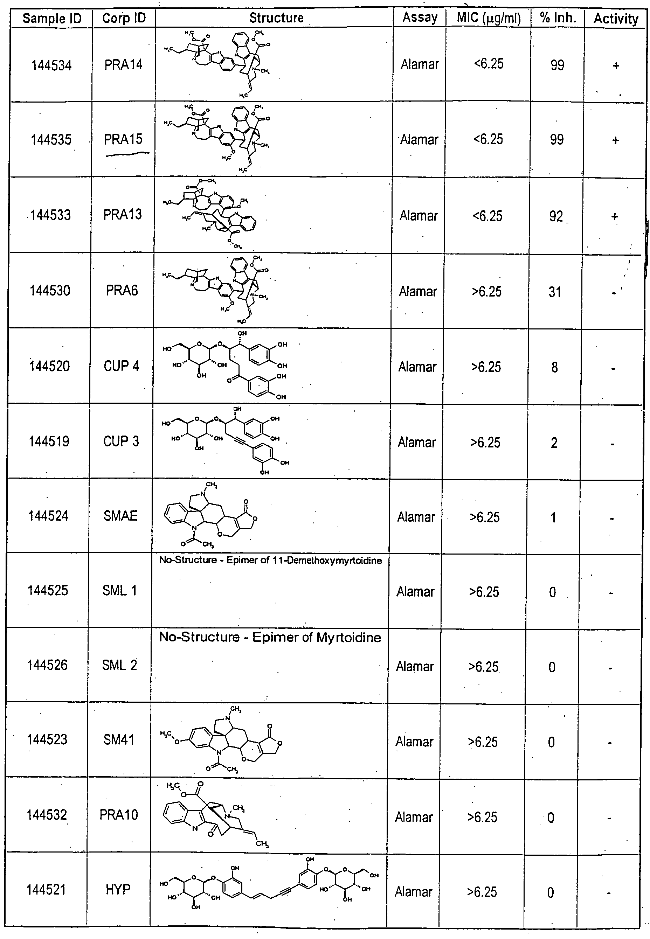

## Revendications

1. Voacamina caractérisée comme agent j'active, stimulante modulante et donc renforçant du système immunitaire.

2. Agent stimulant et modulant et donc renforçant un système immunitaire en second lieu revendication 1).

3. Usage des parties extraites ou composantes de Peschiera Fuchsiaefolia et/ou de Voacanga Africaine, comme d'agent stimulant et modulant et donc renforçant du système immunitaire humain sous forme d'un extrait basique de plante comprenant la Voacamina, caractérisé d'un extrait basique et un extrait de Peschiera Fuchsiaefolia et/ou de Voacanga Africaine en second lieu les revendication 1) - 2)

4. Agent, seconde la revendication 3), qui l'extrait sont un extrait de Peschiera Fuchsiaefolia et/ou de Voacanga Africaine comme agent stimulant et modulant et donc renforçant de range immunitaire.

5. Agent, seconde les revendication 4), caractérisé dans si parce que l'extrait basique est un extrait de l'écorce, et/ou en particulier de l'écorce des racines de Peschiera Fuchsiaefolia et/ou de la Voacanga Africaine.

6. Agent, seconde les revendication 5), caractérisé dans l'extrait basique et une fraction riche en alcaloïdes terziari.

7. Agent, seconde le revendication 6), caractérisé dans si parce qu'il comprend ensemble à la Voacamina, au moins une des suivants alcaloïdes : Peverina, 16-epi- affirina, Affinisina, nb- Demethylvoacamina, Vobasina, Voachalotina, Heyneanina, Voacristina ou Voacangerina, Conopharyngina, Coronadina et Voacangina.

8. Composition pharmaceutique vous contenez des parties, extraits ou des composantes de Peschiera Fuchsiaefolia et/ou de Voacanga Africaine ou un de le sien, physiologiquement acceptables.

9. Composition pharmaceutique vous contenez des parties, extraits ou des composantes de Peschiera Fuchsiaefolia et/ou Voacanga Africaine, ou d'un de le sien tu montes physiologiquement acceptables avec ou sans ajoutée d'excipients physiologiquement acceptables, second les revendications 1-2-3-4-5-6-7-8).

10. Composition pharmaceutique vous contenez des parties, extraits ou composantes de Peschiera Fuchsiaefolia et/ou Voacanga Africaine, sous forme de bases libres ou de sels physiologiquement acceptables associé à un supporte avec ou sans ajoutée d'excipients physiologiquement acceptables.

11. Procédure d'isolation d'un agent stimulant et modulant et donc renforçant du système immunitaire en second lieu revendications 1-2-3-4-5-6-7-8-9-10), caractérisé pour le fait qui comprend-les suivantes étapes constituantes : à
a) traitement avec un acide dilué de l'extrait sec de Peschiera Fuchsiaefolia et/ou de Voacanga Africaine ou d'une partie seule de la Plante pour extraire les principes bases nous.
b) Faire change l'état alcaline de l'extrait aqueux ainsi obtenu à un PH9 et extraire la fraction riche en alcaloïdes terziari avec un solvant organique en particulier du Dichloromethano.

12. Procédure d'isolation de la Voacamina à partir de l'extrait basique en second lieu le revendication 11) pour distribution dans "contre - courant" entre une phase aqueuse et une phase organique en faisant varier PH de la phase aqueuse pour passages suivants, la Voacamina se récupère à PH3,2.

13. Procédure d'isolation seconda la revendication 12), caractérisée du fait qui la plante de départ est une planta du genre Peschiera en particulier Peschiera Fuchsiaefolia et/ou du Voacanga Africaine.
